(19) Europäisches Patentamt
European Patent Office
Office européen des brevets

(11) Veröffentlichungsnummer: **0 021 076**
**B1**

(12) # EUROPÄISCHE PATENTSCHRIFT

(45) Veröffentlichungstag der Patentschrift: **23.03.83**

(51) Int. Cl.³: **A 01 N 43/64**

(21) Anmeldenummer: **80102942.2**

(22) Anmeldetag: **27.05.80**

(54) **Verfahren zur Regulierung des Pflanzenwachstums.**

(30) Priorität: **26.06.79 DE 2925687**

(43) Veröffentlichungstag der Anmeldung:
**07.01.81 Patentblatt 81/1**

(45) Bekanntmachung des Hinweises auf die Patenterteilung:
**23.03.83 Patentblatt 83/12**

(84) Benannte Vertragsstaaten:
**AT BE CH DE FR GB IT LI LU NL SE**

(56) Entgegenhaltungen:
**DE-A-2 720 949**

(73) Patentinhaber: **BASF Aktiengesellschaft,
Carl-Bosch-Strasse 38, D-6700 Ludwigshafen (DE)**

(72) Erfinder: **Zeeh, Bernd, Dr., Thorwaldsenstrasse 5,
D-6700 Ludwigshafen (DE)**
Erfinder: **Buschmann, Ernst, Dr., An der Froschlache 7,
D-6700 Ludwigshafen (DE)**
Erfinder: **Jung, Johann, Dr., Hardenburgstrasse 19,
D-6703 Limburgerhof (DE)**

**EP 0 021 076 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Regulierung des Pflanzenwachstums

Die vorliegende Erfindung betrifft Mittel zur Regulierung des Pflanzenwachstums, die Triazolylglykolether enthalten.

Es ist bekannt, daß 2-Chlorethyltrimethylammoniumchlorid, CCC (J. Biol. Chem. 235, 475 (1960)) pflanzenwachstumsregulierende Eigenschaften bei Getreide und anderen Kulturpflanzen aufweist. Bei der Anwendung entsprechender Mittel zur Regulierung des Pflanzenwachstums ist die Wirkung vor allem bei niedrigen Aufwandmengen und Konzentrationen jedoch oft nicht ausreichend.

Es ist ferner bekannt, Triazolylglykolether zur Bekämpfung von Pilzen im Boden oder an Pflanzen zu verwenden (DE-A-2 720 949). Hierdurch wird die Anwendung zur Regulierung des Pflanzenwachstums bei speziellen Pflanzen nicht nahegelegt.

Es ist ferner bekannt, Triazolylallylether zur Regulierung des Pflanzenwachstums zu verwenden (DE-OS 2 650 831). Außerdem ist auch die Eignung von Phenyl-butylketonen, die durch Triazol substituiert sind, zur Regulierung des Pflanzenwachstums bekannt (DE-OS 2 739 352).

Es wurde nun gefunden, daß Triazolylglykolether der Formel

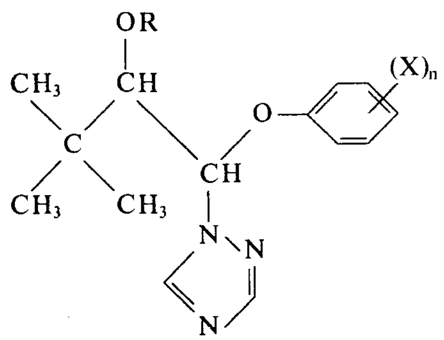

(I)

in der R einen Alkylrest (z. B. Methyl, Ethyl, Propyl, Butyl), einen Cycloalkylrest, einen Alkenylrest (z. B. Allyl, Crotyl, 3-Methyl-2-buten-(1)-yl) oder einen Alkinylrest (z. B. Propargyl), wobei die Reste R durch 1 bis 3 Halogenatome substituiert sein können, X Wasserstoff, Alkyl (z. B. Methyl, Ethyl, t-Butyl) oder Halogen (Fluor, Chlor, Brom, Jod) und n die Zahlen 1 bis 3 bedeutet und die Salze dieser Verbindungen, insbesondere die pflanzenverträglichen Salze (z. B. Hydrochloride, Hydrobromide, Sulfate, Nitrate, Oxalate, Acetate), eine sehr gute Wirkung zur Regulierung des Wachstums von Raps, Soja oder Baumwolle zeigen. R bedeutet beispielsweise: Methyl, Ethyl, Propyl, Isopropyl, n-Butyl, Isobutyl, 1-Methylpropyl, n-Pentyl, 1-Methyl-pentyl, 2-Methyl-pentyl, 3-Methyl-pentyl, 4-Methyl-pentyl, Allyl, Crotyl, Methallyl, Propargyl, Butin-2-yl, 2,3-Dichlorallyl, 2,3,3-Trichlorallyl, Cyclohexyl, Cyclopentyl.

X bedeutet beispielsweise: Wasserstoff, Chlor, Fluor, Brom, Jod.

Die Wirkstoffe greifen in den Metabolismus der Pflanzen ein und sind deshalb als Wachstumsregulatoren geeignet.

Für die Wirkungsweise von Pflanzenwachstumsregulatoren gilt nach der bisherigen Erfahrung, daß ein Wirkstoff eine oder mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden.

Wichtig ist auch die Anwendung von Wachstumsregulatoren zur Hemmung des Längenwachstums zum Beispiel bei Soja, denn durch eine Stengeleinkürzung wird die Gefahr des Umknickens (»Lagerns«) der Pflanzen vor der Ernte verringert oder vollkommen beseitigt.

Eine Hemmung des vegetativen Wachstums erlaubt bei vielen Kulturpflanzen eine dichtere Anpflanzung der Kultur, so daß ein Mehrertrag, bezogen auf die Bodenfläche, erzielt werden kann.

Ein weiterer Mechanismus der Ertragssteigerung mit Wuchshemmern beruht darauf, daß die Nährstoffe in stärkerem Maße der Blüten- und Fruchtbildung zugute kommen, während das vegetative Wachstum eingeschränkt wird.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, so daß z. B. mehr oder größere Früchte zur Ausbildung kommen.

Erstragssteigerungen können in manchen Fällen auch durch einen Eingriff in den pflanzlichen Stoffwechsel erreicht werden, ohne daß sich Änderungen des vegetativen Wachstums bemerkbar

machen. Wachstumsregulatoren können ferner eine Veränderung der Zusammensetzung der Pflanzen bewirken, um so eine bessere Qualität der Ernteprodukte herbeizuführen. So ist es beispielsweise möglich, den Proteingehalt in Soja zu steigern. Unter dem Einfluß von Wachstumsregulatoren kann es zur Ausbildung parthenokarper Früchte kommen. Ferner kann das Geschlecht der Blüten beeinflußt werden.

Mit Wachstumsregulatoren läßt sich auch die Produktion oder der Abfluß von sekundären Pflanzenstoffen positiv beeinflussen.

Während des Wachstums der Pflanze kann durch Einsatz von Wachstumsregulatoren auch die seitliche Verzweigung durch eine chemische Brechung der Apikaldominanz vermehrt werden. Daran besteht z. B. Interesse bei der Stecklingsvermehrung von Pflanzen. Es ist jedoch auch möglich, das Wachstum der Seitentriebe zu hemmen.

Unter dem Einfluß von Wachstumsregulatoren kann der Blattbestand von Pflanzen so gesteuert werden, daß ein Entblättern der Pflanzen zu einem gewünschten Zeitpunkt erreicht wird. Eine derartige Entlaubung ist von Interesse, um eine mechanische Beerntung, z. B. bei Baumwolle, zu erleichtern oder um die Transpiration zu einem Zeitpunkt herabzusetzen, an dem die Pflanze verpflanzt werden soll.

Durch Einsatz von Wachstumsregulatoren läßt sich der vorzeitige Fruchtfall verhindern. Es ist jedoch auch möglich, den Fruchtfall, im Sinne einer chemischen Ausdünnung bis zu einem bestimmten Ausmaß zu fördern. Wachstumsregulatoren können auch dazu dienen, um bei Kulturpflanzen zum Zeitpunkt der Ernte die zum Ablösen der Früchte erforderliche Kraft zu vermindern, so daß eine mechanische Beerntung der Pflanzen ermöglicht bzw. eine manuelle Beerntung erleichtert wird.

Mit Wachstumsregulatoren läßt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Hilfe von Wachstumsregulatoren auch eine zeitliche Konzentrierung der Reife erzielt werden.

Durch Anwendung von Wachstumsregulatoren kann auch die Samen- oder Knospenruhe der Pflanzen, also die endogene Jahresrhythmik, beeinflußt werden, so daß die Pflanzen zu einem Zeitpunkt keimen, austreiben oder blühen, an dem sie normalerweise hierzu keine Bereitschaft zeigen.

Mit Wachstumsregulatoren kann auch erreicht werden, daß der Austrieb von Knospen oder die Keimung von Samen verzögert wird, z. B. um in frostgefährdeten Gebieten eine Schädigung durch Spätfrost zu vermeiden.

Wachstumsregulatoren können auch eine Halophilie bei Kulturpflanzen erzeugen. Damit werden die Voraussetzungen dafür geschaffen, daß eine Kultivierung von Pflanzen auf salzhaltigen Böden durchgeführt werden kann.

Mit Wachstumsregulatoren kann auch eine Frost- und Trockenresistenz induziert werden.

Die Wirkstoffe sind z. T. bekannt aus der DE-OS-2 720 949. Man erhält sie beispielsweise, wenn man die aus DE-OS-2 324 010 bekannten Triazolylglykole der Formel II

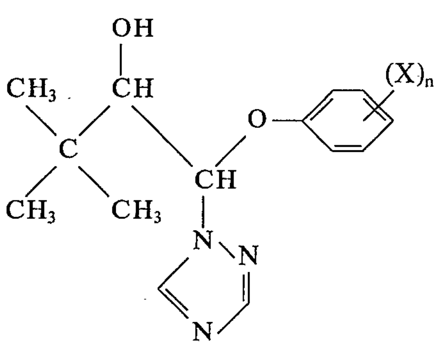

(II)

in welcher X und n die obengenannten Bedeutungen haben, in Gegenwart von Basen in Lösungsmitteln mit Alkylierungsmitteln oder Formel III

$$R—X \qquad (III)$$

in der R die obengenannten Bedeutungen hat und X für eine abspaltbare Gruppe, wie z. B. Halogen,

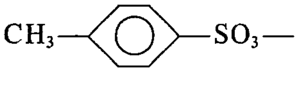 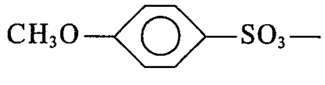 $OSO_3R$

steht, umsetzt. Gut geeignet für diese Umsetzung ist auch die Phasentransfer-Methode (E.V. Dehmlow, Angew. Chem. 89, 521 – 533 (1977)).

Die folgende Vorschrift erläutert die Herstellung der Verbindungen der Formel I.

Zu einer Lösung von 14,8 g 1-(4-Chlorphenoxy)-1-(1,2,4-triazol-1-yl)-3,3-dimethyl-butan-2-ol und 9 g Tetrabutylammoniumhydrogensulfat in 100 ml 1,2,3-Trichlorpropen werden 50 g 50%ige (Gew.-%) wäßrige NaOH zugetropft. Dabei wird die Temperatur durch Eiskühlung unter 30° C gehalten. Nach 24 Stunden Rühren bei Raumtemperatur (20° C) werden 150 ml Ether und 50 ml Wasser zugegeben. Die organische Phase wird dreimal mit 50 ml Wasser gewaschen, mit $Na_2SO_4$ getrocknet und eingeengt. Das ölige Rohprodukt wird in $CH_2CL_2$ aufgenommen und über Kieselgel chromatographiert. Man erhält 7,2 g farbloses Öl. Das Produkt hat folgende Strukturformel

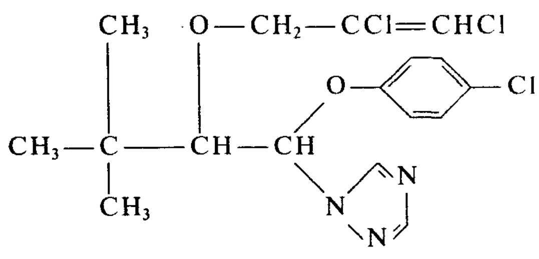

Elementaranalyse:

| | | | | | |
|---|---|---|---|---|---|
| ber.: | C 50,5 | H 5,0 | O 7,9 | N 10,4 | Cl 26,3 |
| gef.: | C 50,5 | H 5,0 | | N 10,1 | Cl 26,3 |

Das NMR-Spektrum ($CDCl_3$, δ-Werte) zeigt zwei Diastereomere:

Isomer 1: 0,90 (s, 9H), 3,78 (d, J = 3 Hz, 1H), 4,62 (d, J = 12 Hz, 1H) 5,83 (d, J = 12 Hz, 1H) 6,42 (d, J = 3 Hz, 1H), 6,43 (s, 1H), 6,82 (d, J = 9 Hz, 2H), 7,21 (d, J = 9 Hz, 2H), 7,96 (s, 1H), 8,63 (s, 1H)

Isomer 2: 0,90 (s, 9H) 3,74 (d, J = 3 Hz, 1H), 4,38 (d, J = 12 Hz, 1H), 4,52 (d, J = 12 Hz, 1H), 6,37 (d, = 3 Hz, 1H), 6,41 (s, 1H), 6,82 (d, J = 9 Hz, 2H), 7,21 (d, J = 9 Hz, 2H), 7,97 (s, 1H), 8,56 (s, 1H)

Beispiele für andere Wirkstoffe, die analog obiger Vorschrift hergestellt werden können, zeigt die folgende Tabelle.

Die in der Tabelle aufgeführten Substanzen ergaben korrekte Elementaranalysen. Eine zusätzliche Charakterisierung erfolgte durch $^1$H-NMR-Spektren.

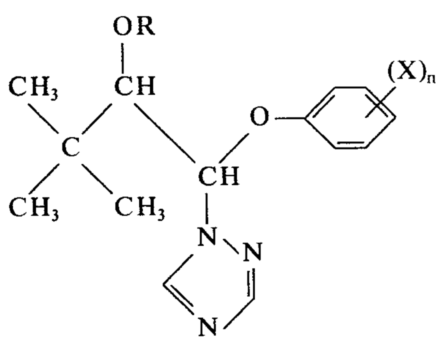

| Nr. | R | (X)n | Schmp. °C |
|---|---|---|---|
| 1 | $CH_3$ | 4-Cl | Öl |
| 2 | $C_2H_5$ | 4-Cl | Öl |
| 3 | n-$C_3H_7$ | 4-Cl | Öl |
| 4 | n-$C_4H_9$ | 4-Cl | 60–62 |
| 5 | $CH_2$-$CH(CH_3)_2$ | 4-Cl | Öl |
| 6 | n-$C_5H_{11}$ | 4-Cl | |
| 7 | $CH_2CHCH_3CH_2CH_2CH_3$ | 4-Cl | |
| 8 | $CH_2CH_2CH(CH_3)_2$ | 4-Cl | |
| 9 | $CH_2-CH=CH_2$ | 4-Cl | Öl |
| 10 | $CH_2-CH=CH-CH_3$ | 4-Cl | Öl |
| 11 | $CH_2CCH_3=CH_2$ | 4-Cl | Öl |
| 12 | $CH_2-CH_2CH=CH_2$ | 4-Cl | |
| 13 | $CH_2CCH_3=CHCH_3$ | 4-Cl | |
| 14 | $CH_2CH=C(CH_3)_2$ | 4-Cl | |
| 15 | $CH_2C{\equiv}CH$ | 4-Cl | Öl |
| 16 | $CH_2-C{\equiv}CCH_3$ | 4-Cl | Öl |
| 17 | $CH_2CCl=CHCl$ | 4-Cl | Öl |
| 18 | $CH_3$ | 2,4-Cl | 153 |
| 19 | $CH_3$ | 2-Cl | Öl |
| 20 | $CH_2CH=CH_2$ | 2-Cl | Öl |
| 21 | $CH_3$ | 4-Br | Öl |
| 22 | $CH_2CH=CH_2$ | 4-Br | 157 |
| 23 | $CH_3$ | H | |
| 24 | $CH_2CH=CH_2$ | H | |
| 25 | $CH_2CCl=CHCl$ | H | |
| 26 | $CHCH_3CH=CH_2$ | 4-Cl | Öl |
| 27 | $CH_3$ | 4-F | |

Die erfindungsgemäßen Mittel können in den üblichen Formulierungen vorliegen, wie Lösungen, Emulsionen, Suspensionen, Stäube, Pulver, Pasten und Granulate.

Die Anwendungsformen richten sich ganz nach den Verwendungszwecken, sie sollen in jedem Fall eine feine gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische

Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Äthanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel, wie nicht ionische und anionische Emulgatoren (z. B. Polyoxyäthylen — Fettalkohol — Äther, Alkylsulfonate und Arylsulfante) und Dispergiermittel, wie Lignin-, Sulfitablaugen und Methylcellulose.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die Mittel enthalten 0,5 bis 95%, vorzugsweise 1 bis 90%, Wirkstoff. Die Aufwandmengen betragen 0,01 bis 10 kg Wirkstoff je ha, vorzugsweise 0,1 bis 5 kg/ha.

Beispiel 1

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel 2

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 10 000 Gewichtsteilen Wasser erhält man eine wäßrige Dispersion, die 0,02 Gew.-% des Wirkstoffs enthält.

Beispiel 3

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

Beispiel 4

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

Beispiel 5

20 Gewichtsteile des Wirkstoffs werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

Beispiel 6

3 Gewichtsteile der Verbindung 3 werden mit 97 Gewichtsteilen feinteiligem Kaolin innig vermischt. Man erhält auf diese Weise ein Stäubemittel, das 3 Gew.-% des Wirkstoffs enthält.

Beispiel 7

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 8

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.-% Wirkstoff enthält.

Beispiel 9

20 Teile des Wirkstoffs 2 werden mit 2 Teilen Calciumsalz der Dodecylbenzolsulfonsäure, 8 Teilen Fettalkoholpolyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Zur Bestimmung der wachstumsregulierenden Eigenschaft der Wirkstoffe wurden Testpflanzen auf ausreichend mit Nährstoffen versorgtem Boden in Kunststoffgefäßen von ca. 12,5 cm Durchmesser angezogen.

Im Vorauflaufverfahren wurden die Testsubstanzen in wäßriger Aufbereitung am Tage der Einsaat auf das Saatbett gegossen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanzen dienten drei bekannte Wirkstoffe.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen.

Vergleichssubstanzen:

Kurzbezeichnung des Wirkstoffs

CCC 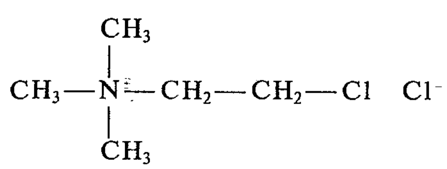

A 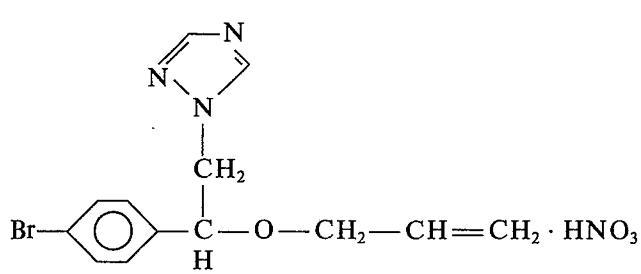

bekannt aus DE-OS 2 650 831

B 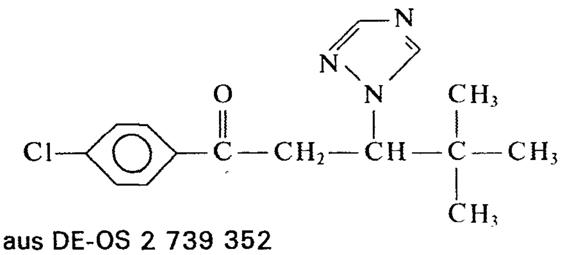

bekannt aus DE-OS 2 739 352

Sommerraps »Cosa«

Vorauflaufverfahren; Versuchsdauer: 21 Tage (WS = Wirkstoff)

| Wirkstoff | Konz. mg WS/gef. | Wuchshöhen cm | % |
|---|---|---|---|
| Unbehandelt | – | 19,8 | 100 |
| CCC | 3 | 17,5 | 88,4 |
| | 12 | 16,5 | 83,3 |
| A | 3 | 18,5 | 93,4 |
| | 12 | 13,5 | 68,2 |
| B | 3 | 18,0 | 90,2 |
| | 12 | 14,0 | 70,7 |
| 16 | 3 | 17,0 | 85,9 |
| | 12 | 12,0 | 60,6 |

Sommerraps »Petronova«

Nachauflaufverfahren; Versuchsdauer: 21 Tage

| Wirkstoff | Konz. mg WS/gef. | Wuchshöhen cm | % |
|---|---|---|---|
| Unbehandelt | – | 22,1 | 100 |
| CCC | 1,5 | 21,0 | 95,0 |
| | 6 | 21,0 | 95,0 |
| A | 1,5 | 20,0 | 90,5 |
| | 6 | 18,5 | 83,7 |
| B | 1,5 | 20,0 | 90,5 |
| | 6 | 18,0 | 81,4 |
| 1 | 1,5 | 18,5 | 83,7 |
| | 6 | 16,0 | 72,4 |
| 9 | 1,5 | 19,5 | 88,2 |
| | 6 | 15,5 | 70,1 |

Sommerraps »Cosa«

Nachauflaufverfahren; Versuchsdauer: 21 Tage

| Wirkstoff | Konz. | Wuchshöhen | |
|---|---|---|---|
| | mg WS/gef. | cm | % |
| Unbehandelt | – | 20,3 | 100 |
| CCC | 1,5 | 18,0 | 88,7 |
| | 6 | 17,0 | 83,7 |
| B | 1,5 | 17,5 | 86,2 |
| | 6 | 16,5 | 81,3 |
| 3 | 1,5 | 17,5 | 86,2 |
| | 6 | 13,5 | 66,5 |
| 4 | 1,5 | 16,5 | 81,3 |
| | 6 | 15,0 | 73,9 |
| 10 | 1,5 | 14,0 | 69,0 |
| | 6 | 11,0 | 54,2 |
| 11 | 1,5 | 15,5 | 76,4 |
| | 6 | 12,5 | 61,6 |
| 26 | 1,5 | 15,0 | 73,9 |
| | 6 | 11,5 | 56,7 |

Sojabohnen »SRF 450«

Nachauflaufverfahren; Versuchsdauer: 29 Tage

| Wirkstoff | Konz. | Wuchshöhen | |
|---|---|---|---|
| | mg WS/gef. | cm | % |
| Unbehandelt | – | 26,3 | 100 |
| CCC | 1,5 | 24,0 | 91,3 |
| | 6 | 22,0 | 83,7 |
| A | 1,5 | 25,0 | 95,1 |
| | 6 | 21,0 | 79,9 |
| B | 1,5 | 26,0 | 98,9 |
| | 6 | 25,5 | 97,0 |
| 1 | 1,5 | 20,0 | 76,1 |
| | 6 | 17,0 | 64,6 |
| 9 | 1,5 | 23,0 | 87,5 |
| | 6 | 20,0 | 76,1 |

Sojabohnen »SRF 450«

Nachauflaufverfahren; Versuchsdauer: 38 Tage

| Wirkstoff | Konz. | Wuchshöhen | |
|---|---|---|---|
| | mg WS/gef. | cm | % |
| Unbehandelt | – | 25,3 | 100 |
| CCC | 1,5 | 25,0 | 98,8 |
| | 6 | 22,0 | 87,0 |
| B | 1,5 | 25,0 | 98,8 |
| | 6 | 22,0 | 87,0 |
| 15 | 1,5 | 19,0 | 75,1 |
| | 6 | 16,5 | 65,2 |
| 18 | 1,5 | 22,0 | 87,0 |
| | 6 | 21,0 | 83,0 |
| 21 | 1,5 | 20,0 | 79,1 |
| | 6 | 17,5 | 69,2 |
| 22 | 1,5 | 22,5 | 88,9 |
| | 6 | 19,0 | 75,1 |

Baumwolle »Delta Pine«

Nachauflaufverfahren; Versuchsdauer 27 Tage

| Wirkstoff | Konz. | Wuchshöhen | |
|---|---|---|---|
| | mg WS/gef. | cm | % |
| Unbehandelt | – | 27,6 | 100 |
| B | 1,5 | 27,5 | 99,6 |
| | 3 | 27,0 | 97,8 |
| 1 | 1,5 | 26,0 | 94,2 |
| | 3 | 24,0 | 87,0 |
| 9 | 1,5 | 24,0 | 87,0 |
| | 3 | 24,0 | 87,0 |
| 15 | 1,5 | 25,0 | 90,6 |
| | 3 | 20,0 | 72,5 |
| 21 | 1,5 | 25,0 | 90,6 |
| | 3 | 24,0 | 87,0 |
| 22 | 1,5 | 25,0 | 90,6 |
| | 3 | 22,5 | 81,5 |

**Patentansprüche**

1. Verfahren zur Regulierung des Pflanzen-Wachstums, dadurch gekennzeichnet, daß man Pflanzen von Raps, Soja oder Baumwolle behandelt mit einem Triazolylglykolether der Formel

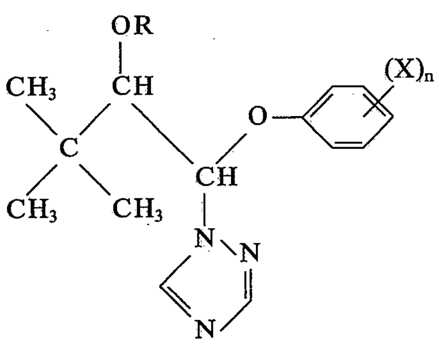

(I)

in der

R einen Alkyl-, Cycloalkyl-, Alkenyl- oder Alkinylrest, die durch 1 bis 3 Halogenatome substituiert sein können,
X Wasserstoff, Halogen oder Alkyl und
n die Zahlen 1 bis 3 bedeutet

oder einem Salz dieser Verbindung.

2. Verfahren nach Anspruch 1, dadurch gekennzeichnet, daß man für die Behandlung einen Triazolylglykolether, ausgewählt aus der Gruppe, bestehend aus

1-(4-Chlorphenoxy)-3,3-dimethyl-2-methoxy-1-(1,2,4-triazol-1-yl)-butan,
1-(4-Bromphenoxy)-3,3-dimethyl-2-methoxy-1-(1,2,4-triazol-1-yl)-butan,

2-Allyloxy-1-(4-chlorphenoxy)-3,3-dimethyl-1-(1,2,4-triazol-1-yl)-butan,
2-(Buten-2-oxy)-1-(4-chlorphenoxy)-3,3-dimethyl-1-(1,2,4-triazol-1-yl)-butan und
2-(Butin-2-oxy)-1-(4-chlorphenoxy)-3,3-dimethyl-1-(1,2,4-triazol-1-yl)-butan

verwendet.

**Claims**

1. A process for regulating plant growth, wherein rape, soybean or cotton plants are treated with a triazolyl glycol ether of the formula

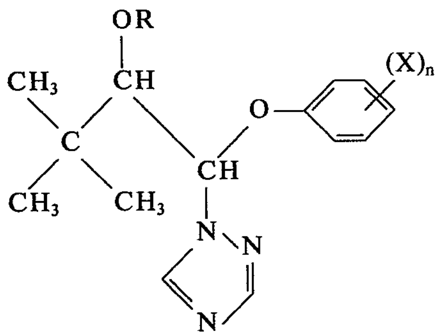

(I)

where R denotes alkyl, cycloalkyl, alkenyl or alkynyl which are unsubstituted or substituted by from 1 to 3 halogen atoms, X denotes hydrogen, halogen or alkyl, and n denotes one of the integers 1, 2 and 3, or a salt thereof.

2. A process as claimed in claim 1, wherein the triazolyl glycol ether used for the treatment is selected from the group consisting of

1-(4-chlorophenoxy)-3,3-dimethyl-2-methoxy-1-(1,2,4-triazol-1-yl)-butane,
1-(4-bromophenoxy)-3,3-dimethyl-2-methoxy-1-(1,2,4-triazol-1-yl)-butane,
2-allyloxy-1-(4-chlorophenoxy)-3,3-dimethyl-1-(1,2,4-triazol-1-yl)-butane,
2-(buten-2-oxy)-1-(4-chlorophenoxy)-3,3-dimethyl-1-(1,2,4-triazol-1-yl)-butane, and
2-(butyn-2-oxy)-1-(4-chlorophenoxy)-3,3-dimethyl-1-(1,2,4-triazol-1-yl)-butane.

**Revendications**

1. Procédé pour la régulation de la croissance de plantes, caractérisé en ce que l'on traite les plantes de colza, de soja ou de coton avec un éther de triazolyl-glycol de la formule

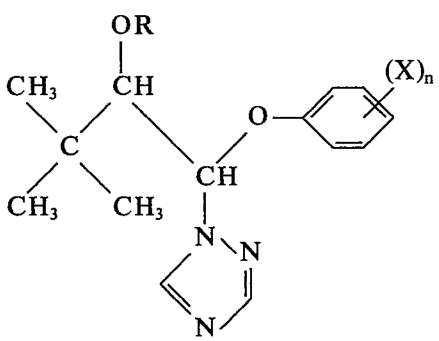

(I)

dans laquelle

R désigne un radical alcoyle, cyclo-alcoyle, alcényle ou alcynyle pouvant être substitué par un à trois atomes d'halogène,
X est un atome d'hydrogène ou d'halogène ou un radical alcoyle et
n est un nombre valant de 1 à 3,

ou avec un sel d'un tel composé.

2. Procédé suivant la revendication 1, caractérisé en ce que le traitement est réalisé avec un éther de triazolyl-glycol choisi parmi

le 1-(4-chlorophénoxy)-3,3-diméthyl-2-méthoxy-1-(1,2,4-triazol-1-yl)-butane,
le 1-(4-bromophénoxy)-3,3-diméthyl-2-méthoxy-1-(1,2,4-triazol-1-yl)-butane,
le 2-allyloxy-1-(4-chlorophénoxy)-3,3-diméthyl-1-(1,2,4-triazol-1-yl)-butane,
le 2-(butène-2-oxy)-1-(4-chlorophénoxy)-3,3-diméthyl-1-(1,2,4-triazol-1-yl)-butane et
le 2-(butyne-2-oxy)-1-(4-chlorophénoxy)-3,3-diméthyl-1-(1,2,4-triazol-1-yl)-butane.